(19) Europäisches Patentamt
European Patent Office
Office européen des brevets

(11) Numéro de publication: **0 403 318 A1**

(12) **DEMANDE DE BREVET EUROPEEN**

(21) Numéro de dépôt: **90401237.4**

(22) Date de dépôt: **10.05.90**

(51) Int. Cl.5: **C07D 233/88, A61K 35/56, A61K 31/415**

(30) Priorité: **12.05.89 FR 8906251**

(43) Date de publication de la demande: **19.12.90 Bulletin 90/51**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Ahond, Alain**
**66 rue Galliéni**
**F-92240 Malakoff(FR)**
Inventeur: **Almourabit, Ali**
**Karl Schurz Weg 4B**
**D-3000 Hannover(DE)**
Inventeur: **Bedoya, Zurita Manuel**
**7 rue Bailly**
**F-75003 Paris(FR)**
Inventeur: **Potier, Pierre**
**14 avenue de Breteuil**
**F-75007 Paris(FR)**
Inventeur: **Poupat, Christiane**
**11 rue des Peupliers**
**F-78370 Plaisir(FR)**
Inventeur: **Commercon, Alain**
**1 bis rue Charles Floquet**
**F-94400 Vitry Sur Seine(FR)**

(74) Mandataire: **Pilard, Jacques et al**
**RHONE-POULENC SANTE, Service Brevets,**
**20 Avenue Raymond Aron**
**F-92165 Antony Cédex(FR)**

(54) **Procédé de préparation d'une nouvelle substance antitumorale, l'amino-3 (amino-2 1H-imidazolyl-4-)-1 chloro-2 propanol-1 et de ses sels sous forme racémique thréo, utilisable comme médicament.**

(57) Le racémique thréo de l'amino-3 (amino-2 1H-imidazolyl-4)-1 chloro-2 propanol-1 de formule générale :

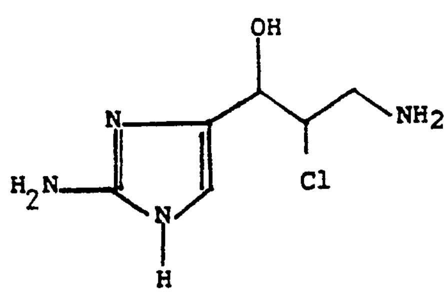

ses sels, son procédé de préparation et les compositions pharmaceutiques qui le contiennent.

EP 0 403 318 A1

# PROCEDE DE PREPARATION D'UNE NOUVELLE SUBSTANCE ANTITUMORALE, L'AMINO-3 (AMINO-2 1H-IMIDAZOLYL-4)-1 CHLORO-2 PROPANOL-1 ET DE SES SELS SOUS FORME RACEMIQUE THREO, UTILISABLE COMME MEDICAMENT

La présente invention concerne un procédé de préparation d'une nouvelle substance antitumorale, l'amino-3 (amino-2 1H-imidazolyl-4)-1 chloro-2 propanol-1 et de ses sels sous forme racémique thréo (1RS,2RS).

Dans le brevet français FR 85 10997 (2 585 020) a été décrite une substance biologiquement active, désignée sous le nom de girolline, extraite d'une éponge identifiée à Pseudaxinyssa cantha rella, qui présente des propriétés antitumorales remarquables.

La structure de la girolline, sous forme de base ou de sel, peut être représentée par la formule suivante :

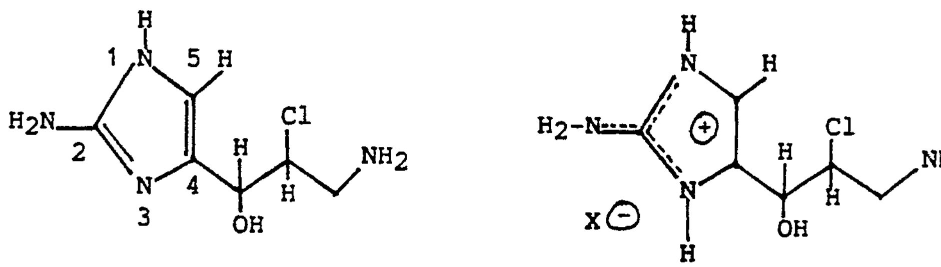

(I)

dans laquelle $X^-$ représente un anion tel que l'anion chlorure ($Cl^-$).

Un produit correspondant à la structure donnée ci-dessus, compte tenu de la présence de deux atomes de carbone asymétriques, peut exister sous forme de 4 isomères qui correspondent aux énantiomères des racémiques érythro et thréo.

Les études effectuées sur la girolline montrent que le produit d'origine naturelle correspond à l'un des énantiomères du racémique thréo.

Il a également été montré que le racémique thréo conserve les propriétés antitumorales remarquables de la girolline et que le racémique érythro est totalement inactif.

La présente invention a pour objet un procédé de préparation du racémique thréo du produit de formule (I).

Selon la présente invention, le racémique thréo du produit de formule (I) peut être obtenu par la réduction, suivie d'une acidification d'un dérivé azo sous forme de racémique thréo de formule générale :

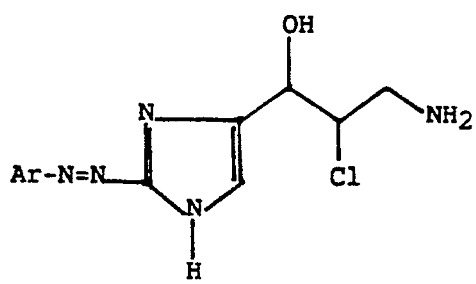

(II)

dans laquelle Ar représente un radical phényle éventuellement substitué par un ou plusieurs radicaux, identiques ou différents, choisis parmi les atomes d'halogène (fluor, chlore, brome) et les radicaux alcoyles contenant 1 à 4 atomes de carbone, alkoxycarbonyles dont la partie alkyle contient 1 à 4 atomes de carbone, et nitro.

Généralement, la réduction est effectuée au moyen d'hydrogène en présence d'un catalyseur tel que l'oxyde de platine en opérant dans un solvant organique tel qu'un alcool (méthanol, éthanol) à une température comprise entre 0 et 30 °C. Il n'est pas nécessaire d'opérer sous pression.

L'acidification est effectuée au moyen d'un acide minéral dilué tel que l'acide chlorhydrique.

Le produit racémique thréo de formule générale (II) peut être obtenu par action d'un sel de diazonium de formule générale :

$Ar\text{-}N_2^+ , X^-$ (III)

dans laquelle Ar est défini comme précédemment et $X^-$ représente un anion, tel qu'un ion halogénure ou tétrafluoroborate, éventuellement préparé in situ, sur un produit racémique thréo de formule :

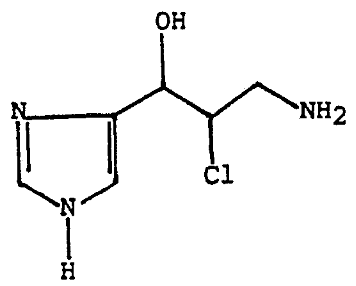

(IV)

La diazotation est généralement effectuée à une température voisine de 0 °C en opérant dans une solution aqueuse basique diluée, telle qu'une solution aqueuse de carbonate de sodium.

Le produit racémique thréo de formule (IV) peut être obtenu par traitement en milieu acide d'un produit racémique thréo de formule générale :

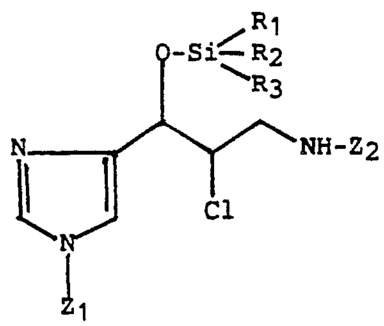

(V)

dans laquelle $R_1$, $R_2$, $R_3$, identiques ou différents, représentent des radicaux alkyles contenant 1 à 4 atomes de carbone ou phényles, $Z_1$ représente un groupement protecteur de la fonction amine éliminable en milieu acide tel qu'un radical trityle, tert.butoxycarbonyle, diméthylsulfamoyle ou camphosulfonyle et $Z_2$ représente un radical alkoxycarbonyle tel que le radical tert.butoxycarbonyle. De préférence, le radical $-SiR_1R_2R_3$ est un radical tert.butyldiméthylsilyle ou diphényltert.butylsilyle.

Généralement l'élimination des groupements protecteurs est effectuée par chauffage au reflux dans un acide minéral dilué tel que l'acide chlorhydrique.

Le produit racémique thréo de formule générale (V) peut être obtenu par halogénation d'un produit racémique érythro de formule générale :

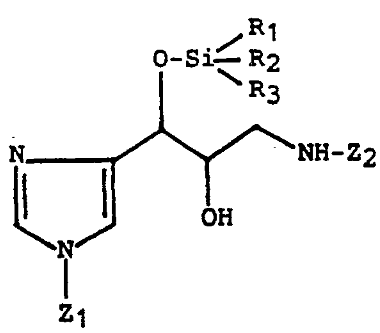

(VI)

dans laquelle $R_1$, $R_2$, $R_3$, $Z_1$ et $Z_2$ sont définis comme précédemment.

De préférence, la réaction est effectuée par la triphénylphosphine dans le tétrachlorure de carbone au reflux.

Le produit de formule générale (VI) sous forme racémique érythro est obtenu après séparation du mélange des formes racémiques érythro et thréo qui est lui-même obtenu par réaction d'oxyamination d'un

produit de formule générale :

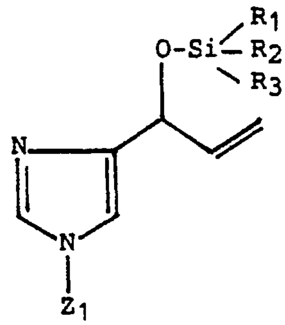

(VII)

dans laquelle $R_1$, $R_2$, $R_3$ et $Z_1$ sont définis comme précédemment au moyen du sel de sodium d'un N-chlorocarbamate d'alkyle de formule générale :

$Z_2$-NH-Cl (VIII)

dans laquelle $Z_2$ est défini comme précédemment, préparé in situ en opérant dans un solvant organique tel que l'acétonitrile, le benzène, le toluène, en présence d'eau et en présence d'un sel minéral tel que le nitrate d'argent, le chlorure de zinc, le chlorure mercurique et d'une solution tert.butanolique de tétroxyde d'osmium.

Les racémiques érythro et thréo peuvent être séparés de leur mélange par chromatographie sur un support approprié, tel que la silice en éluant avec un solvant organique convenable.

Le produit de formule générale (VII) peut être obtenu par silylation d'un alcool de formule générale :

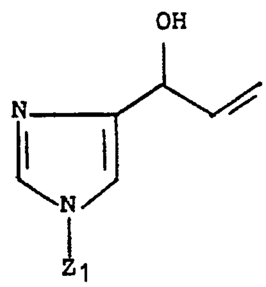

(IX)

dans laquelle $Z_1$ est défini comme précédemment, au moyen d'un halogénosilane de formule générale :

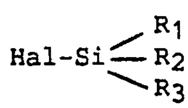

(X)

dans laquelle $R_1$, $R_2$ et $R_3$ sont définis comme précédemment et Hal représente un atome d'halogène en opérant dans un solvant organique tel que le diméthylformamide.

Le produit de formule générale (IX) peut être obtenu par action du bromure de vinylmagnésium sur l'aldéhyde de formule générale :

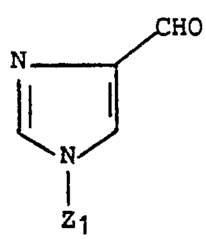

(XI)

dans laquelle $Z_1$ est défini comme précédemment, suivie de l'hydrolyse du magnésien obtenu.

Le produit racémique thréo de formule générale (II) peut aussi être obtenu par hydrolyse acide du

produit racémique thréo de formule générale :

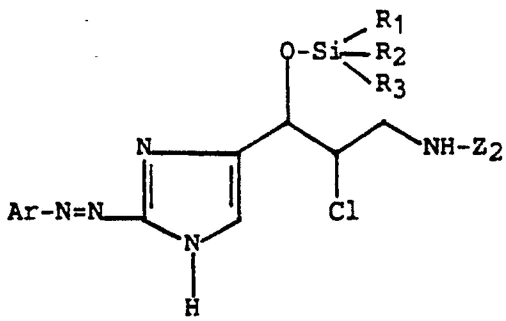

(XII)

dans laquelle Ar, $R_1$, $R_2$, $R_3$ et $Z_2$ sont définis comme précédemment.

Généralement l'hydrolyse est effectuée au moyen d'acide chlorhydrique dans le méthanol.

Le produit racémique thréo de formule générale (XII) peut être obtenu par action d'un sel de diazonium de formule générale (III) sur un produit racémique thréo de formule générale :

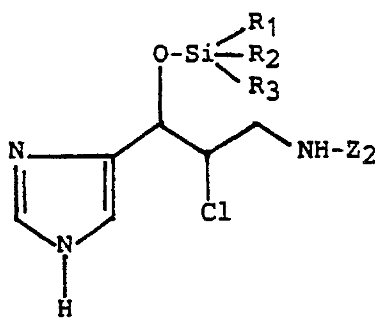

(XIII)

dans laquelle $R_1$, $R_2$, $R_3$ et $Z_2$ sont définis comme précédemment, dans les conditions décrites précédemment pour la réaction d'un produit de formule générale (III) sur un produit de formule générale (IV).

Le produit de formule générale (XIII) peut être obtenu par élimination sélective du groupement protecteur $Z_1$ d'un produit de formule générale (V). Par exemple, lorsque $Z_1$ représente un radical trityle, le remplacement de ce radical par un atome d'hydrogène s'effectue par chauffage dans un alcool tel que le n.propanol. Dans le but d'accélérer la réaction, il peut être avantageux d'opérer en présence d'un acide organique tel que l'acide acétique.

Le racémique thréo du produit de formule (I) présente l'avantage d'être plus facilement accessible que la girolline elle-même sans pour autant présenter des inconvénients qui pourraient nuire à son utilisation thérapeutique humaine en tant que médicament.

La présente invention concerne également les compositions pharmaceutiques qui contiennent le racémique thréo du produit de formule (I) en association avec tout autre produit pharmaceutiquement acceptable, qu'il soit inerte ou physiologiquement actif.

Les compositions peuvent être présentées sous toute forme appropriée à la voie d'administration prévue. La voie parentérale est la voie d'administration préférentielle et notamment la voie intra-veineuse.

Les compositions selon l'invention pour administration parentérale peuvent être des solutions stériles aqueuses ou non aqueuses, des suspensions ou des émulsions comme solvant ou véhicule, on peut employer le propylèneglycol, les huiles végétales, en particulier l'huile d'olive, et les esters organiques injectables, par exemple l'oléate d'éthyle. Ces compositions peuvent également comprendre des adjuvants en particulier des agents mouillants, émulsifiants ou dispersants. La stérilisation peut se faire de plusieurs façons, par exemple a l'aide d'un filtre bactériologique, en incorporant à la composition des agents stérilisants, par irradiation ou par chauffage. Elles peuvent également être préparées sous forme de compositions solides stériles qui peuvent être dissoutes ou dispersées au moment de l'emploi dans de l'eau stérile ou tout autre milieu stérile injectable.

Le racémique thréo du produit de formule (I) est particulièrement utile dans le traitement des hémopathies malignes et des tumeurs solides à des doses journalières généralement comprises entre 0,1 et 1 mg/kg par voie intraveineuse pour un adulte.

Les exemples suivants, donnés à titre non limitatif, montrent comment l'invention peut être mise en

pratique.

EXEMPLE 1 - Préparation de l'amino-3 (amino-2 1H-imidazolyl-4)-1 chloro-2 propanol-1-(1RS,2RS)

On dissout 60 mg d'amino-3 [(bromo-4 phénylazo)-2 1H-imidazolyl-4]-1 chloro-2 propanol-1-(1RS,2RS) (0,17 mmole) dans 10 cm3 de méthanol. On hydrogène à passion ordinaire en présence d'une quantité catalytique de $PtO_2$. Le mélange réactionnel est agité sous atmosphère d'hydrogène pendant 24 heures, puis filtré sur célite. Après évaporation à sec du filtrat, le produit obtenu est trituré avec 20 cm3 d'éther pour éliminer la bromo-4 aniline. Après décantation, la phase éthérée est éliminée. Le résidu est repris par 1 cm3 d'acide chlorhydrique 2N. L'insoluble est séparé par filtration puis lavé avec 2 x 1cm3 d'eau. Le filtrat et les eaux de lavage réunis sont concentrés à sec. On obtient ainsi 29 mg d'un produit brut qui est chromatographié sur une colonne de silice (60 Merck, 0,040-0,063 mm) de 10 de diamètre en éluant avec un mélange acétate d'éthyle- méthyléthylcétone-acide formique-eau (5-3-1-1 en volumes). On obtient ainsi 4,5 mg d'amino-3 (amino-2 1H-imidazolyl-4)-1 chloro-2 propanol-1-(1RS,2RS) sous forme de dichlorhydrate.

Les autres fractions obtenues ainsi que l'insoluble isolé précédemment sont soumis au même processus d'hydrogénation et de purification. On obtient ainsi, au total, 9,5 mg d'amino-3 (amino-2 1H-imidazolyl-4)-1 chloro-2 propanol-1-(1RS,2RS) sous forme de dichlorhydrate.

Ce produit est dissous dans 0,5 cm3 de méthanol puis filtré sur une colonne de Sephadex LH20 (diamètre : 10 mm ; hauteur : 20 cm) en éluant au méthanol.

Après évaporation du solvant, on obtient 8 mg d'amino-3 (amino-2 1H-imidazolyl-4)-1 chloro-2 propanol-1-(1RS,2RS) sous forme de dichlorhydrate ayant un pouvoir rotatoire nul et dont la structure est confirmée par comparaison avec le produit naturel par le spectre de masse, le spectre de résonance magnétique nucléaire du proton et la chromatographie en couche mince dans différents systèmes de solvants.

Le rendement est de 18 %.

EXEMPLE 2 - Préparation de l'amino-3 [(bromo-4 phénylazo)-2 1H-imidazolyl-4]-1 chloro-2 propanol-1-(1RS,2RS)

On ajoute une solution refroidie à 0° C de 107 mg (1,56 m.mole) de nitrite de sodium dans 1 cm3 d'eau à une solution refroidie à 0° C de 264 mg (1,53 m.mole) de bromo-4 aniline dans 6 cm3 d'acide chlorhydrique 2N. On maintient le mélange à 0° C pendant 40 minutes puis on ajoute goutte à goutte la solution du sel de diazonium obtenue à 300 mg de chlorhydrate d'amino-3 (1H-imidazolyl-4)-1 chloro-2 propanol-1-(1RS,2RS) (1,41 mmole) en solution dans 30 cm3 d'une solution aqueuse de carbonate de sodium à 10 % (p/v) refroidie à 0° C. Un précipité rouge-orangé apparaît. Après 15 minutes d'agitation à 0° C, on neutralise par addition d'acide chlorhydrique concentré puis laisse au repos pendant 2 heures à 4° C.

Le précipité obtenu est séparé par filtration, lavé avec 3 fois 10 cm3 d'eau puis repris par 100 cm3 d'un mélange chlorure de méthylène-méthanol (9-1 en volumes). La solution est évaporée à sec pour donner un produit brut.

La phase aqueuse est extraite par 3 fois 20 cm3 de n-butanol saturé d'eau. Les phases n-butanoliques sont concentrées à sec. Le produit ainsi obtenu, réuni au produit brut obtenu précédemment, est chromatographié sur une colonne de silice (60 Merck ; 0,040-0,063 mm) (diamètre : 30 mm) en éluant avec un mélange chlorure de méthylène-méthanol (8-2 en volumes).

On obtient ainsi, avec un rendement de 12 %, 61 mg d'amino-3 [(bromo-4 phénylazo)-2 1H-imidazolyl-4]-1 chloro-2 propanol-1- (1RS,2RS) sous forme d'une poudre amorphe dont les caractéristiques sont les suivantes :

- Rf = 0,3 (chlorure de méthylène-méthanol ; 8-2 en volumes)
- spectre ultra-violet : $\lambda max$ = 203 nm ($\epsilon$ = 9750)
$\lambda max$ = 242 nm ($\epsilon$ = 5100)
$\lambda max$ = 384 nm ($\epsilon$ = 11460)
- spectre infra-rouge : principales bandes d'absorption caractéristiques à 1565, 1020 et 830 $cm^{-1}$
- spectre de masse (FAB, glycérol, mode positif) : pic à m/z = 358.
- spectre de résonance magnétique nucléaire du proton (400 MHz, $CD_4O$ à 3,33 ppm, $\delta$ en ppm, J en Hz) : 3,16 (m 1H) ; 3,32 (m, 1H) ; 4,22 (m, 1H) ; 4,80 (d, J = 4, 1H) ; 6,70 (s, 1H) ; 7,03 (d, J = 8, 2H) ; 7,14 (d, J = 8, 2H).

EXEMPLE 3 - Préparation de l'amino-3 chloro-2 (1H-imidazolyl-4)-1 propanol-1-(1RS,2RS)

a) On chauffe au reflux pendant 3 heures, 800 mg (1,27 m.mole) de N-tert.butoxycarbonyl tert.butyldiméthylsilyloxy-3 chloro-2 (trityl-1 1H-imidazolyl-4)-3 propanamine-1-(2RS,3RS) dans 20 cm3 d'acide chlorhydrique 2N. Le précipité qui se forme (triphénylcarbinol) est séparé par filtration et lavé par 3 fois 10 cm3 d'eau. Les solutions aqueuses réunies sont extraites par 3 fois 10 cm3 d'éther puis concentrées à sec. On obtient ainsi, avec un rendement quantitatif, 312 mg d'amino-3 chloro-2 (1H-imidazolyl-4)-1 propanol-1-(1RS,2SR) sous forme de chlorhydrate dont les caractéristiques sont les suivantes :

- Rf = 0,32 (acétate d'éthyle-méthyléthylcétone-acide formique-eau ; 5-3-2-2 en volumes ; révélation en bleu par le système eau de Javel-o.tolidine)
- spectre ultra-violet : λmax = 205 nm (ϵ = 5210)
- spectre de masse : (FAB, glycérol, mode positif) : pic à m/z = 176
- spectre de résonance magnétique nucléaire du proton (400 MHz, $D_2O$ à 4,83 ppm, δ en ppm, J en Hz) : 3,47 (dd, J = 14 et 10, 1H) ; 3,65 (dd, J = 14 et 3, 1H) ; 4,65 (d épais, J = 10, 1H) ; 5,41 (s épais, 1H) ; 7,54 (s, 1H) ; 8,73 (s, 1H)
- spectre de résonance magnétique nucléaire du $^{13}C$ (50 MHz ; $D_2O$ ; référence interne : dioxanne à 67,8 ppm, δ en ppm) : 44,2 ; 61,3 ; 67,4 ; 118,1 ; 133 ; 135,3

b) En opérant comme dans l'exemple 3a) mais en utilisant le N-tert.butoxycarbonyl chloro-2 diphényltert.butylsilyloxy-3 (trityl-1 1H-imidazolyl-4)-3 propanamine-1-(2RS,3RS), on obtient le chlorhydrate d'amino-3 chloro-2 (1H-imidazolyl-4-)-1 propanol-1-(1RS,2RS) identique à celui obtenu à l'exemple 3a avec un rendement de 87 %.

EXEMPLE 4 - Préparation du N-tert.butoxycarbonyl tert.butyldiméthylsilyloxy-3 chloro-2 (trityl-1 1H-imidazolyl-4)-3 propanamine-1-(2RS,3RS)

A une solution de 1,0 g (1,63 m.mole) de tert.butoxycarbonylamino-3 tert.butyldiméthylsilyloxy-1 (trityl-1 1H-imidazolyl-4)-1 propanol-2-(1RS,2SR) dans 15 cm3 de tétrachlorure de carbone anhydre, on ajoute, sous atmosphère d'argon, 1,28 g (4,9 m.moles) de triphénylphosphine préalablement recristallisée dans l'hexane et séchée en présence d'anhydride phosphorique à 65 °C sous pression réduite (1 mm de mercure ; 0,13 kPa). Le mélange réactionnel est chauffé lentement jusqu'au reflux. On maintient au reflux pendant 24 heures. Après évaporation à sec, le résidu est chromatographié sur une colonne de silice (60 Merck ; 0,040-0,063 mm) (diamètre ; 60 mm) en éluant avec un mélange hexane-acétate d'éthyle (7-3 en volumes). On obtient ainsi avec un rendement quantitatif 1,02 g de N-tert.butoxycarbonyl tert.butyldiméthylsilyloxy-3 chloro-2 (trityl-1 1H-imidazolyl-4)-3 propanamine-1-(2RS,3RS) dont les caractéristiques sont les suivantes :

- point de fusion : 73-75 °C (après recristallisation dans un mélange acétate d'éthyle-hexane)
- Rf = 0,28 (hexane-acétate d'éthyle : 7-3 en volumes ; révélation en jaune par la vanilline sulfurique)
- spectre ultra-violet : max = 208 nm (ϵ = 34530)
- spectre infra-rouge (en solution dans le chloroforme) : principales bandes d'absorption caractéristiques à 3430, 3140, 2950-2850, 1700, 1485, 1445, 1115, 840 et 700 $cm^{-1}$
- spectre de masse (ic, isobutane, 170 °C) : pics à m/z (%) : 632 (98), 390 (100), 243 (100).
- spectre de résonance magnétique nucléaire du proton (400 MHz, $CDCl_3$, δ en ppm, J en Hz) : - 0,16 (s,3H) ; 0,05 (s,3H) ; 0,79 (s,9H) ; 1,45 (s,9H) ; 3,27 (m,1H) ; 3,64 (m,1H) ; 4,12 (m,1H) ; 4,85 (d,J = 5,1H) ; 5,16 (s,1H) ; 6,77 (s,1H) ; 7,14 (m,6H) ; 7,32 (m,9H) ; 7,38 (s,1H).

EXEMPLE 5 - Préparation du N-tert.butoxycarbonyl chloro-2 diphényl tert.butylsilyloxy-3 (trityl-1 1H-imidazolyl-4)-3 propanamine-1-(2RS,3RS)

En opérant comme dans l'exemple 4, mais en utilisant le tert.butoxycarbonylamino-3 diphényltert.butylsilyloxy-l (trityl-1 1H-imidazolyl-4)-1 propanol-2-(2RS,3SR), on obtient, avec un rendement de 52 %, le N-tert.butoxycarbonyl chloro-2 diphényltert.butylsilyloxy-3 (trityl-1 1H-imidazolyl-4)-3 propanamine-1-(2RS,3SR) présentant les caractéristiques suivantes :

- Rf = 0,70 (héxane-éther ; 3-5 en volumes)
- spectre infra-rouge (en solution dans le chloroforme) : principales bandes caractéristiques à 3410, 3050-2840, 1700, 1490, 1440, 1360, 1260, 1160, 1120, 1110, 900 et 695 $cm^{-1}$
- spectre de masse (i.c., 190 °C) : pic à m/z (%) = 758, 756 (79), 378 (49), 260, 258 (72), 243 (100)
- spectre de résonance magnétique nucléaire du proton (200 MHz, $CDCl_3$, δ en ppm, J en Hz) = 1,0 (s,9H)

; 1,42 (s,9H) ; 3,67 (m,2H) ; 4,27 (d, J = 5,1H) ; 4,80 (d, J = 5,1H) ; 4,88 (m,1H) ; 6,50 (s,1H); 7,0-7,63 (m,26H).

EXEMPLE 6 - Préparation du tert.butoxycarbonylamino-3 tert.butyldiméthylsilyloxy-1 (trityl-1 1H-imidazolyl-4)-1 propanol-2-(1RS,2SR)

A une solution de 2 g (17 m.moles) de tert.butylcarbamate dans 15 cm3 de méthanol, on ajoute à 0° C, 1,91 cm3 (1 équivalent) d'hypochlorite de tert.butyle. Après 15 minutes d'agitation, on ajoute goutte à goutte, à 0° C, une solution de 0,68 g (1 équivalent) de soude dans 9 cm3 de méthanol. La solution est agitée, à une température voisine de 20° C, pendant 10 minutes. Après distillation du solvant à une température voisine de 20° C, le résidu blanc obtenu est trituré avec 30 cm3 d'éther anhydre puis séparé par filtration, lavé avec 3 fois 10 cm3 d'éther anhydre. On obtient ainsi le sel de sodium du N-chlorocarbamate de tert.butyle qui est conservé sous atmosphère d'argon à -18° C en présence de chlorure de calcium.

Dans un ballon de 100 cm3, on introduit 1,04 g (6 m.moles) du sel de sodium du N-chlorocarbamate de tert.butyle, 40 cm3 d'acétonitrile et 2,04 g (12 m.moles) de nitrate d'argent. On agite pendant 5 minutes et, à la suspension jaune-pâle obtenue, on ajoute 0,32 cm3 d'eau distillée, 1,92 g (4 m.moles) de (tert.butyldiméthylsilyloxy-1 propène-2 yl-1)-4 trityl-1 1H-imidazole (R,S) et 0,4 cm3 (0,04 m.mole) d'une solution de tétroxyde d'osmium ($OsO_4$) à 2,5 % (p/v) dans le tert.butanol. Après 20 heures d'agitation à une température voisine de 20° C, on ajoute 1 cm3 d'une solution saturée de chlorure de sodium. Le précipité obtenu est séparé par filtration sur célite. Après concentration à sec du filtrat, on obtient 2,9 g d'un produit brut qui est chromatographié sous forme de pâte sur une colonne de silice (60 Merck ; 0,040-0,063 mm) (diamètre : 60 mm) en éluant avec un mélange hexane-acétate d'éthyle (6-4 en volumes). On obtient ainsi 1,45 g de tert.butoxycarbonylamino-3 tert.butyldiméthylsilyloxy-1 (trityl-1 1H-imidazolyl-4)-1 propanol-2-(1RS,2SR) avec un rendement de 59 % et 0,34 g de tert.butoxycarbonylamino-3 tert.butyldiméthylsilyloxy-1 (trityl-1 1H-imidazolyl4)-1 propanol-2-(1RS,2RS) avec un rendement de 15 %.

Le tert.butoxycarbonylamino-3 tert.butyldiméthylsilyloxy-1 (trityl-1 1H-imidazolyl-4)-1 propanol-2-(1RS,2SR) présente les caractéristiques suivantes :

- point de fusion : 51-53° C (après recristallisation dans le mélange acétate d'éthyle-hexane)
- Rf = 0,3 (hexane-acétate d'éthyle : 6-4 en volumes ; révélé en jaune par la vanilline sulfurique)
- spectre ultra-violet : λmax = 207 nm (ε = 37290)
- spectre infra-rouge (en solution dans le chloroforme) : principales bandes d'absorption caractéristiques à 3400, 2950-2850, 1700, 1675, 1600, 1490, 1440, 1365, 1250, 840 et 700 $cm^{-1}$
- spectre de masse (ie, 200° C) : pics à m/z (%) : 613 ($M^+$, 11), 243 (100), 228 (64), 212 (97), 165 (100), 153 (99), 75 (76), 73 (68), 57 (68), 41 (36)
- spectre de résonance magnétique nucléaire du proton (400 MHz, $CDCl_3$, δ en ppm, J en Hz) : -0,28 (s, 3H) ; -0,07 (s, 3H) ; 0,68 (s, 9H) ; 1,34 (s, 9H) ; 1,98 (s épais, 1H) ; 3,10 (m, 1H) ; 3,32 (m, 1H) ; 3,77 (m, 1H) ; 4,60 (d, J = 5, 1H) ; 5,13 (s épais, 1H) ; 6,72 (s, 1H) ; 7,12 (m, 6H) ; 7,34 (m, 9H) ; 7,40 (s, 1H).

Le tert.butoxycarbonylamino-3 tert.butyldiméthylsilyloxy-1 (trityl-1 1H-imidazolyl-4)-1 propanol-2-(1RS,2RS) présente les caractéristiques suivantes :

- point de fusion : 48-50° C (après recristallisation dans le mélange chlorure de méthylène-hexane)
- Rf = 0,45 (hexane-acétylacétate d'éthyle : 6-4 en volumes ; révélation par la vanilline sulfurique)
- spectre infra-rouge (en solution dans le chloroforme) : principales bandes d'absorption caractéristiques à 3550-3425, 3000-2850, 1700, 1680, 1600, 1500, 1260-1210, 1170, 1140, 840 et 700 $cm^{-1}$
- spectre de masse (ie, 200° C) : pics à m/z (%) : 613 ($M^+$, <1), 243 (100), 228 (22), 211 (44), 165 (100)
- spectre de résonance magnétique nucléaire du proton (400 MHz, $CDCl_3$, δ en ppm, J en Hz) : -0,14 (s, 3H) ; 0,02 (s, 3H) ; 0,77 (s, 9H) ; 1,43 (s, 9H) ; 2,02 (s épais, 1H) ; 3,0 (m, 1H) ; 3,35 (m, 1H) ; 3,83 (m, 1H) ; 4,72 (d, J = 4, 1H) ; 5,07 (s épais, 1H) ; 6,72 (s, 1H) ; 7,17 (m, 6H) ; 7,32 (m, 9H) et 7,38 (s, 1H).

EXEMPLE 7 - Préparation du tert.butoxycarbonylamino-3 diphényltert.butylsilyloxy-1 (trityl-1 1H-imidazolyl-4)-1 propanol-2-(1R5,2SR)

En opérant comme dans l'exemple 6, mais en utilisant le (diphényltert.butylsilyloxy-1 propène-2 yl-1)-4 trityl-1 1H-imidazole-(R,S), on obtient, un mélange des racémiques érythro (1RS,2SR) (53 %) et thréo (1RS,2RS) (14 %) du tert.butoxycarbonylamino-3 diphényltert.butylsilyloxy-1 (trityl-1 1H-imidazolyl-4)-1 propanol-2 dont on sépare les constituants par chromatographie.

Le racémique érythro (1RS,2SR) est caractérisé par :
- Rf = 0,30 (hexane-éther ; 2-8 en volumes)
- spectre infra-rouge (en solution dans le chloroforme) : principales bandes d'absoption caractéristiques à 3450-3200, 3100-2850, 1720, 1500, 1370, 1250, 1170, 1120 et 910 $cm^{-1}$
- le spectre de masse (ic, 200 °C) : pic à m/z (%) = 738 (58), 496 (87), 257(98), 243 (98), 240 (100), 167 (100)
- le spectre de résonance magnétique nucléaire du proton (400 MHz, $CDCl_3$, δ en ppm, J en Hz) : 1,0 (s, 9H) ; 1,42 (s, 9H) ; 3,03 (m, 1H) ; 3,40 (m, 1H) ; 4,11 (m, 1H) ; 4,58 (d, J = 5, 1H) ; 5,02 (m, 1H) ; 6,37 (sep, W1/2 = 4,1H) ; 6,94 - 7,59 (26H).

Le racémique thréo (1RS,2RS) est caractérisé par :
-Rf = 0,40 (hexane-éther ; 2-8 en volumes)
- spectre infra-rouge (en solution dans le chloroforme) : principales bandes d'absorption caractéristiques à 3400-3200, 3050-2850, 1700, 1490, 1365, 1240, 1165, 1110, 700, 910 et 700 $cm^{-1}$
- le spectre de masse (ic, 200 °C) : pic à m/z (%) = 738 (45), 496 (100), 257(100), 243 (100), 240 (100), 167 (98)
-le spectre de résonance magnétique nucléaire du proton (400 MHz, $CDCl_3$, δ en ppm, J en Hz) : 1,0 (s, 9H) ; 1,42 (s, 9H) ; 3,08 (m, 1H) ; 3,40 (m, 1H) ; 3,93 (m, 1H) ; 4,65 (d, J = 4,1H) ; 5,04 (m, 1H) ; 6,33 (s, 1H), 6,93-7,57 (m, 26H).

EXEMPLE 8 - Préparation du (tert.butyldiméthylsilyloxy-1 propène-2 yl-1)-4 1H-imidazole

Dans un ballon de 25 cm3, on introduit, sous atmosphère d'argon, 1,02 g (15 m.moles) d'imidazole, 8 cm3 de diméthylformamide et 0,905 g (6 m.moles) de chlorure de tert.butyldiméthylsilyle. La solution est agitée pendant 15 minutes à une température voisine de 20 °C puis refroidie à 0 °C. On ajoute 1,83 g (5 m.moles) de (trityl-1 1H-imidazolyl-4)-1 propène-2 ol-1. Après 1 heure d'agitation à une température voisine de 20 °C, le mélange réactionnel devient limpide. La solution est versée sur 300 g de glace. Le précipité blanc pâteux obtenu est séparé par filtration, lavé 3 fois par 50 cm3 d'eau puis repris par 100 cm3 de chlorure de méthylène. La solution chlorométhylénique est séchée sur sulfate de magnésium, filtrée puis concentrée à sec. On obtient ainsi, avec un rendement quantitatif, 2,39 g de (tert.butyldiméthylsilyloxy-1 propène-2 yl-1)-4 trityl-1 1H-imidazole sous forme d'une poudre amorphe dont les caractéristiques sont les suivantes :
- Rf = 0,4 (hexane-acétate d'éthyle : 7-3 en volumes ; révélation en jaune par la vanilline sulfurique)
- spectre ultra-violet : λmax = 220 nm (ε = 11060)
λmax = 260 nm (ε = 610)
- spectre infra-rouge (en solution dans le chloroforme) : principales bandes d'absorption caractéristiques à 3150, 3050, 2950, 1590, 1485, 1440, 1250, 905, 840 et 700 $cm^{-1}$
- spectre de masse (ic, isobutane, 200 °C) : pics à m/z (%) : 481 (100), 243 (100), 239 (47), 133 (17), 107 (28)
- spectre de résonance magnétique nucléaire du proton (400 MHz, $CDCl_3$, δ en ppm, J en Hz) : 0,06 (s, 3H) ; 0,14 (s, 3H) ; 0,93 (s, 9H) ; 5,23 (d, J = 10, 1H) ; 5,32 (d, J = 5, 1H) ; 5,44 (d, J = 17, 1H) ; 6,18 (ddd, J = 17, 10 et 5, 1H) ; 6,83 (s, 1H) ; 7,23 (m, 6H); 7,41 (m, 9H) et 7,45 (s, 1H).

EXEMPLE 9 - Préparation du (trityl-1 1H-imidazolyl-4)-1 propène-2 ol-1

Dans un ballon de 250 cm3 préchauffé, on introduit, sous atmosphère d' argon, 50 cm3 de tétrahydrofuranne anhydre et 12 cm3 d'une solution 1M de bromure de vinylmagnésium (12 m.moles) dans le tétrahydrofuranne. Après refroidissement à 0 °C, on ajoute goutte à goutte une solution de 3,38 g (10 m.moles) de trityl-1 1H-imidazolecarboxaldéhyde-4 dans 50 cm3 de tétrahydrofuranne anhydre. Après une nuit d'agitation à une température voisine de 20 °C, on ajoute une solution aqueuse concentrée contenant 0,805 g (15 m.moles) de chlorure d'ammonium. On agite pendant 1 heure. Le précipité formé est séparé par filtration et lavé 3 fois avec 30 cm3 de tétrahydrofuranne. Le filtrat est concentré à sec. Le résidu est chromatographié sur une colonne de silice (60 Merck ; 0,040-0,063 mm) (diamètre : 70 mm) en éluant avec un mélange hexane-acétate d'éthyle (2-8 en volumes). On obtient ainsi, avec un rendement de 94 %, 3,42 g de (propène-2 yl-1)-4 trityl-1 1H-imidazole dont les caractéristiques sont les suivantes :
- point de fusion : 153-155 °C (après recristallisation dans un mélange chlorure de méthylène-hexane)
- Rf = 0,4 (hexane-acétate d'éthyle : 2-8 en volumes ; révélation en jaune par la vanilline sulfurique)

- spectre ultra-violet : max = 213 ($\epsilon$ = 24500)
- spectre infra-rouge (en solution dans le chloroforme) : principales bandes d'absorption caractéristiques à 3590-3160, 3060, 3000, 1600, 1495, 1445, 1305, 1135, 910 et 700 $cm^{-1}$
- spectre de masse (ie, 180° C) : pics à m/z (%) : 366 (<1), 243 (100), 165 (98)
- spectre de résonance magnétique nucléaire du proton (400 MHz, $CDCl_3$, $\delta$ en ppm, J en Hz) : 5,20 (m, 2H) ; 5,40 (d, J = 17, 1H) ; 6,12 (ddd, J = 17, 10 et 6, 1H) ; 6,74 (s, 1H) ; 7,13 (m, 6H) ; 7,33 (m, 9H) et 7,35 (s, 1H).

Le trityl-1 1H-imidazolecarboxaldéhyde-4 peut être préparé selon le procedé décrit par J.M. KOKOSA et al., J. Org. Chem., 48, 3605 (1983).

EXEMPLE 10 - Préparation du (diphényltert.butylsilyloxy-1 propène-2 yl-1)-4 trityl-1 1H-imidazole

En opérant comme dans l'exemple 8, mais en utilisant le chlorure de diphényltert.butylsilyle et en opérant en présence de 3 équivalents d'imidazole, on obtient, après purification, le (diphényltert.butylsilyloxy-1 propène-2 yl-1)-4 trityl-1 1H-imidazole avec un rendement de 89 %. Ses caractéristiques sont les suivantes :
- Rf = 0,43 (hexane-acétate d'éthyle : 8-2 en volumes)
- spectre infra-rouge (en solution dans le chloroforme) : principales bandes d'absorption caractéristiques à 3025, 2950, 2910, 2850, 1480, 1465, 1440, 1120, 1100, 1020, 900 et 805 $cm^{-1}$
- spectre de masse (ic, à 190° C) : pics à m/z (%) : 605 (44) ; 257 (71) ; 243 (100), 167 (64) ; 107 (100)
- spectre de résonance magnétique nucléaire du proton (200 MHz, $CDCl_3$, $\delta$ en ppm, J en Hz) : 0,98 (s, 9H) ; 5,08 (d, J = 10, 1H) ; 5,15 (m, 2H) ; 6,06 (ddd, J = 16, 10 et 6, 1H) ; 6,57 (s, 1H) ; 6,28 et 7,03 (2m, 22H) ; 7,50 et 7,54 (2d, J = 7, 2H).

EXEMPLE 11 - Préparation de l'amino-3 [(bromo-4 phénylazo)-2 1H-imidazolyl-4]-1 chloro-2 propanol-1-(1RS,2RS)

On agite pendant 6 jours, à une température proche de 20° C, 56 mg de N-tert.butoxycarbonyl [(bromo-4 phénylazo)-2 1H-imidazolyl-4]-3 tert.butyldiméthylsilyloxy-3 chloro-2 propanamine-1(2RS,3RS) dans un mélange de 2 cm3 d'éthanol et de 2 cm3 d'acide chlorhydrique 2N. Le milieu réactionnel est dilué par 10 cm3 d'eau et extrait par 10 cm3 d'oxyde d'isopropyle. La phase organique est éliminée puis la phase aqueuse est alcalinisée par une solution saturée de bicarbonate de sodium et extraite par 3 fois 10 cm3 de chlorure de méthylène. Les phases organiques réunies sont séchées sur sulfate de magnésium puis concentrées à sec sous pression réduite. On obtient, avec un rendement de 80 %, 27 mg d'un solide jaune ayant les mêmes caractéristiques que le produit décrit dans l'exemple 2.

EXEMPLE 12 - Préparation du N-tert.butoxycarbonyl [(bromo-4 phényl azo)-2 1H-imidasolyl-4]-3 tert.butyldiméthylsilyloxy-3 chloro-2 propanamine-1-(2RS,3RS)

A une solution de 185 mg de N-tert.butoxycarbonyl tert.butyldiméthylsilyloxy-3 chloro-2 (1H-imidasolyl-4)-3 propanamine-1-(2RS,3RS) dans 6 cm3 de tétrahydrofuranne, on ajoute 4,5 cm3 d'eau. On ajoute ensuite à une température proche de 20° C la solution obtenue en dissolvant 103 mg de tétrafluoroborate de bromo-4 phényldiazonium dans 2 cm3 de tétrahydrofuranne. Après 30 minutes d'agitation on ajoute 103 mg de tétrafluoroborate de bromo-4 phényldiazonium supplémentaire en solution dans 2 cm3 de tétrahydrofuranne. Après 30 minutes d'agitation le milieu réactionnel est décanté, la phase aqueuse est extraite par 2 fois 25 cm3 d'oxyde d'isopropyle, les phases organiques sont réunies, séchées sur sulfate de magnésium et concentrées sous pression réduite. Le résidu est chromatographié sur 15 g de silice (Merck, 0,04-0,063 mm) contenue dans une colonne de 2 cm de diamètre. On élue avec un mélange de chlorure de méthylène et d'acétate d'éthyle (7-3 en volumes). Les 25 premiers cm3 sont éliminés, les 100 cm3 suivants sont concentrés à sec sous pression réduite. On obtient, avec un rendement de 31 %, 83 mg de N-tert.butoxycarbonyl tert. butyldiméthylsilyloxy-3 [(bromo-4 phénylazo)-2 1H-imidazolyl-4]-3 chloro-2 propanamine-1-(2RS,3RS) sous forme d'une meringue jaune qui présente les caractéristiques suivantes :
- spectre de résonance magnétique nucléaire du proton (250 MHz, $CDCl_3$, $\delta$ en ppm) : -0,15 (s, 3H) ; 0,1 (s, 3H) ; 0,85 (s épais, 9H) ; 1,40 (s épais, 9H) ; 3,30 (m, 1H) ; 3,65 (m, 1H) ; 4,40 (s épais, 1H) ; 5,20 (s épais, 1H) ; 5,65 (s épais, 1H) ; 7,35 (s épais, 1H) ; 7,60 (d épais, 2H) ; 7,72 (d épais, 2H) ; 7,85 (s épais, 1H).

EXEMPLE 13 - Préparation du N-tert.butoxycarbonyl tert.butyldiméthylsilyloxy-3 chloro-2 (1H-imidazolyl-4)-3 propanamine-1-(2RS,3RS)

Une solution de 3 g de N-tert.butoxycarbonyl tert.butyldiméthylsilyloxy-3 chloro-2 (trityl-1 1H-imidazolyl-4)-3 propanamine-1-(2RS,3RS) dans 150 cm3 de propanol-1 est chauffée au reflux pendant 24 heures. Le mélange réactionnel est alors refroidi, concentré à sec sous pression réduite et le résidu obtenu est chromatographié sur 75 g de silice (Merck, 0,040-0,063 mm) contenue dans une colonne de 3,5 cm de diamètre. On élue avec une solution de chlorure de méthylène et de méthanol (95-5 en volumes). Les 200 premiers cm3 d'éluats sont éliminés puis on concentre à sec sous pression réduite les 160 cm3 suivants. On obtient ainsi, avec un rendement de 75 %, 1,4 g de N-tert.butoxycarbonyl tert.butyldiméthylsilyloxy-3 chloro-2 (1H-imidazolyl-4)-3 propanamine-1-(2RS,3RS) sous forme d'une meringue incolore qui présente les caractéristiques suivantes;
- spectre de résonance magnétique nucléaire du proton (200 MHz, $CDCl_3$, δ en ppm, J en Hz) : -0,07 (s, 3H) ; 0,10 (s, 3H) ; 0,90 (s, 9H) ; 1,46 (s, 9H) ; 3,30 (m, 1H) ; 3,55 (m, 1H) ; 4,15 (qd, J = 6, 1H) ; 4,93 (d, J = 5,5, 1H) ; 7,07 (s, 1H) ; 7,25 (m, 1H) ; 7,70 (s, 1H).

EXEMPLE 14 - Préparation du camphosulfonate d'amino-3 chloro-2 (1H-imidazolyl-4)-1 propanol-1-(1RS,2RS)

On agite pendant une nuit le mélange N-tert.butoxycarbonyl tert.butyldiméthylsilyloxy-3 (camphosulfonyl-(S)-1 1H-imidazolyl-4)-3 chloro-2 propanamine-1-(2RS,3RS), à 60° C, dans l'acide chlorhydrique 2N. On obtient ainsi, avec un rendement quantitatif, le camphosulfonate d'amino-3 chloro-2 (1H-imidazolyl-4)-1 propanol-1-(1RS,2RS) dont la structure est confirmée par le spectre de résonance magnétique nucléaire du proton (400 MHz, $D_2O$ à 4,85 ppm, δ en ppm, J en Hz) : 2,76 - 0,83 (m, 13H) ; 2,88 (d, J = 15, 1H) ; 3,30 (d, J = 15,1H) ; 3,47 (dd, J = 14 et 10,1H) ; 3,63 (dd, J = 14 et 3, 1H) ; 4,67 (d, J = 10, 1H) ; 5,41 (s, 1H) ; 7,55 (s, 1H) ; 7,73 (s, 1H).

EXEMPLE 15 - Préparation de la N-tert.butoxycarbonyl tert.butyldiméthylsilyloxy-3 (camphosulfonyl-(S)-1 1H-imidazolyl-4)-3 chloro-2 propanamine-1-(2RS,3RS)

On opère comme dans l'exemple 4, mais à partir du mélange des isomères (1RS,2RS) et (1RS,2SR) du tert.butoxycarbonylamino-3 tert.butyldiméthylsilyloxy-1 (camphosulfonyl-(S)-1 1H-imidazolyl-4)-1 propanol-2. On obtient ainsi, après purification sur une colonne de silice (60 - 200 µ ; diamètre 60 mm ; hauteur 200 mm), avec l'éluant hexane-acétate d'éthyle (30-70 en volumes) la N-tert.butoxycarbonyl tert.butyldiméthylsilyloxy-3 (camphosulfonyl-(S)-1 1H-imidazolyl-4)-3 chloro-2 propanamine-1 (2RS,3RS) (34 %) et la N-tert. butoxycarbonyl tert.butyldiméthylsilyloxy-3 (camphosulfonyl-(S)-1 1H-imidasolyl-4)-3 chloro-2 propanamine (2SR,3RS) (20 %).

La N-tert. butoxycarbonyl tert.butyldiméthylsilyloxy-3 (camphosulfonyl-(S)-1 1H-imidazolyl-4)-3 chloro-2 propanamine-1 (2RS,3RS) présente les caractéristiques suivantes :
- Rf = 0,43 (hexane-éther : 3-7 en volumes)
- spectre infra-rouge (en solution dans le chloroforme) : principales bandes d'absorption caractéristiques à 3400, 3000-2080, 1730, 1695, 1475, 1435, 1360, 1240 et 1140 $cm^{-1}$
- spectre de masse (ic, à 180° C) : pic à m/z (%) : 606, 604 (69) ; 392, 390 (100)
- spectre de résonance magnétique nucléaire du proton (400 MHz, $CDCl_3$, δ en ppm, J en Hz) : -0,13 (s, 3H) ; 0,77 (s, 3H) ; 0,83 (s, 9H) ; 1,03 (s, 3H) ; 1,33 (s, 9H) ; 1,70-2,45 (m, 7H) ; 3,12 (d, J = 15,1H) ; 3,22 (m, 1H) ; 3,53 (m, 1H) ; 3,68 (d, J = 15,1H) ; 4,15 (m, 1H) ; 4,85 (d, J = 4,1H) ; 5,03 (m, 1H) ; 7,25 (s, 1H) ; 7,88 (s, 1H).

La N-tert. butoxycarbonyl tert.butyldiméthylsilyloxy-3 (camphosulfonyl-(S)-1 1H-imidazolyl-4)-3 chloro-2 propanamine-1 (2SR,3RS) présente les caractéristiques suivantes :
- Rf = 0,49 (hexane-éther : 3-7 en volumes)
- spectre infra-rouge (en solution dans le chloroforme) : principales bandes d'absorption caractéristiques à 3400, 3000-2800, 1735, 1490, 1360, 1240, 1200 et 1160 $cm^{-1}$
- spectre de masse (ic, à 180° C) : pic à m/z (%) : 606, 604 (100) ; 392, 390 (75)
- spectre de résonance magnétique nucléaire du proton (400 MHz, $CDCl_3$, δ en ppm, J en Hz) : -0,12 (s, 3H) ; 0,03 (s, 3H) ; 0,73 (s, 3H) ; 0,82 (s, 9H) ; 1,0 (s, 3H) ; 1,30 (s, 9H) ; 1,65-2,28 (m, 7H); 3,08 (d, J = 15,1H) ; 3,23 (m, 1H) ; 3,42 (m, 1H) ; 3,61 (d, J = 15,1H) ; 4,25 (m, 1H) ; 4,92 (d, J = 3,1H) ; 5,02 (m, 1H) ;

7,27 (s, 1H) ; 7,82 (s, 1H).

EXEMPLE 16 - Préparation du mélange des isomères (1RS,2RS) et (1RS,2SR) du tert.butoxycarbonylamino-3 tert.butyldiméthylsilyloxy-1 [camphosulfonyl-(S)-1 1H-imidazolyl-4]-1 propanol-2

En opérant comme dans l'exemple 6, mais à partir du (tert.butyldiméthylsilyloxy-1 propène-2 yl-1)-4 camphosulfonyl-(S)-1 1H-imidazole et en effectuant la réaction dans le toluène, on obtient avec un rendement de 48 %, le mélange des isomères (1RS,2SR) (62 %) et (1RS,2RS) (18 %) du tert.butoxycarbonylamino-3 tert.butyldiméthylsilyloxy-1 (camphosulfonyl-(S)-1 1H-imidazolyl-4)-1 propanol-2.

Le tert.butoxycarbonylamino-3 tert.butyldiméthylsilyloxy-1 (camphosulfonyl-(S)-1 1H-imidazolyl-4)-1 propanol-2 (1RS,2SR) présente les caractéristiques suivantes :
- Rf = 0,23 (hexane-acétate d'éthyle : 1-1 en volumes)
- spectre infra-rouge (en solution dans le chloroforme) : principales bandes caractéristiques à 3600-3300, 3450, 3050-2850, 1750, 1500, 1390, 1175 et 1080 $cm^{-1}$
- spectre de masse (ie, à 180° C) : pic à m/z (%) : 586, 211 (100) ;
- spectre de résonance magnétique nucléaire du proton (400 MHz, $CDCl_3$, δ en ppm, J en Hz) : 0,02 (s, 3H) ; 0,13 (s, 3H) ; 0,93 (s, 3H) ; 0,96 (s, 9H) ; 1,10 (s, 3H) ; 1,45 (s, 9H) ; 1,78-2,42 (m, 7H); 3,20 (m, 2H) ; 3,37 (m, 1H) ; 3,72 (d, J = 15,1H) ; 3,96 (m, 1H) ; 4,72 (m, 1H) ; 5,10 (m, 1H) ; 7,28 (s, 1H) ; 7,94 (s, 1H).

Le tert.butoxycarbonylamino-3 tert.butyldiméthylsilyloxy-1 (camphosulfonyl-(S)-1 1H-imidazolyl-4)-1 propanol-2 (1RS,2RS) présente les caractéristiques suivantes :
- Rf = 0,24 (hexane-acétate d'éthyle : 1-1 en volumes)
- spectre infra-rouge (en solution dans le chloroforme) : principales bandes caractéristiques à 3450, 3400-2850, 1750, 1700, 1650, 1500, 1380, 1180 et 1080 $cm^{-1}$
- spectre de masse (ie, 190° C) : pic à m/z (%) : 586, 211 (100) ;
- spectre de résonance magnétique nucléaire du proton (400 MHz, $CDCl_3$, δ en ppm, J en Hz) : 0,0 (s, 3H) ; 0,12 (s, 3H) ; 0,88 (s, 3H) ; 0,92 (s, 9H) ; 1,12 (s, 3H) ; 1,43 (s, 9H) ; 1,79-2,40 (m, 7H); 3,03 (m, 1H) ; 3,22 (d, J = 14,5, 1H) ; 3,36 (m, 1H) ; 3,74 (d, J = 14,5, 1H) ; 3,83 (m, 1H) ; 4,73 (d, J = 4, 1H) ; 5,30 (s, 1H) ; 7,26 (s, 1H) ; 7,95 (s, 1H).

EXEMPLE 17 - Préparation du (tert.butyldiméthylsilyloxy-1 propène-2 yl-1)-4 camphosulfonyl-(S)-1 1H-imidazole (1R,S)

En opérant comme dans l'exemple 8, mais à partir du (camphosulfonyl-(S)-1 1H-imidazolyl-4)-1 propène-2 ol-1-(R,S), en présence de 2,9 équivalents d'imidazole, on obtient, avec un rendement de 81 %, le (tertiobutyldiméthylsilyloxy-3 propène-2yl)-4 camphosulfonyl-(S)-1 1H-imidazole (1R,S) dont les caractéristiques sont les suivantes :
- Rf = 0,82 (hexane-acétate d'éthyle : 8-2 en volumes)
- spectre de masse (ie, à 180° C) : pic à m/z (%) : 453, 395 (100) ; 181 (100) ; 151 (43).

EXEMPLE 18 - Préparation du (camphosulfonyl-(S)-1 1H-imidazolyl-4)-1 propène-2 ol-1-(R,S)

On ajoute goutte à goutte 6,2 g de camphosulfonyl-(S)-1 1H-imidazolecarboxaldéhyde-4 en solution dont 100 cm3 de tétrahydrofuranne anhydre à 30 cm3 d'une solution molaire de bromure de vinyl magnésium refroidie à -72° C. Après trente minutes d'agitation,on ajoute une solution de 1,1 g de chlorure d'ammonium dans $30cm^3$ d'eau. Par distillation, on élimine le tétrahydrofuranne; puis on extrait la solution aqueuse par de l'acétate d'éthyle. La phase organique est séchée et distillée à sec : le mélange brut est purifié par chromatographie sur une colonne de silice (60-200 μ) ; diamètre 80 mm ; hauteur 200 mm) avec le mélange hexane-acétate d'éthyle (2-8 en volumes) comme éluant. On obtient 6,4 g de (camphosulfonyl-(S)-1 1H-imidazolyl-4)1 propène-2 ol-1-(R,S) dont les caractéristiques sont les suivantes :
- point de fusion : 110° C (cristallisation de l'éthanol)
- Rf = 0,27 (hexane-acétate d'éthyle : 3-7 en volumes)
- spectre infra-rouge (en solution dans le chloroforme) : principales bandes caractéristiques à 3650-3050, 3050-2900, 1750, 1390, 1185, 1090 et 910 $cm^{-1}$
- spectre de masse (ie, à 180° C) : pics à m/z (%) : 338, 215 (40) ; 150 (40), 123 (60), 64 (100)
- spectre de résonance magnétique nucléaire du proton (400 MHz, $CDCl_3$, δ en ppm, J en Hz) : 0,87 (s, 3H)

; 1,12 (s, 3H) ; 1,50-3,40 (m, 7H) ; 3,22 (d, J = 12,1H) ; 3,74 (d, J = 12,1H) ; 5,23 (d, J = 6,1H) ; 5,25 (d, J = 10,1H) ; 5,47 (d, J = 17,1H) ; 6,12 (m, 1H); 7,31 (s, 1H) ; 7,97 (s, 1H).

- spectre de résonance magnétique nucléaire du carbone (50 MHz, $CDCl_3$, δ en ppm) : 19,5 ; 25,1 ; 26,8 ; 42,2 ; 42,7 ; 48,3 ; 53,9 ; 58,7 ; 68,9 ; 113,8 ; 116,1 ; 136,7 ; 137,9 ; 146,4 ; 213,2

EXEMPLE 19 - Préparation du camphosulfonyl-(S)-1 1H-imidazolecarboxaldéhyde-4

A une solution de sel de potassium de 1H-imidazolecarboxadéhyde-4 préalablement préparé par addition de 7 g de tert.butylate de potassium à 4 g de 1H-imidazolecarboxaldéhyde-4 en solution dans 200 $cm^3$ de tétrahydrofuranne anhydre, on ajoute goutte à goutte 15,37 g de chlorure de l'acide camphosulfonique-(S) en solution dans 50 $cm^3$ de tétrahydrofuranne anhydre. Après une heure, le mélange réactionnel est filtré et le solvant distillé. Le mélange obtenu est redissous dans 500 $cm^3$ de chlorure de méthylène et adsorbé sur 50 g de silice (60-200 µ).

Après distillation du solvant, on place cette pâte sur une colonne de silice (60-200 µ ; diamètre 80 mm ; hauteur 200 mm) et on élue avec un mélange d'hexane-acétate d'éthyle (7-3 en volumes). On récupère 9,5 g de camphosulfonyl-(S)-1 1H-imidazolecarboxaldéhyde-4 (81 %) correspondant aux caractéristiques suivantes :

- point de fusion : 126° C (cristallisation de hexane-acétate d'éthyle)
- Rf = 0,42 (hexane-acétate d'éthyle : 1-1 en volumes)
- spectre ultraviolet : λmax = 244 nm (ε = 4120)
- spectre infra-rouge (en solution dans le chloroforme) : principales bandes caractéristiques à 3150, 3050-2090, 2750, 1760, 1185, 1695, 1535, 1475 et et 1390 $cm^{-1}$
- spectre de masse (ie, à 180° C) : pics à m/z (%) : 310, 215 (100) ; 151 (100), 95 (84)
- spectre de résonance magnétique nucléaire du proton (400 MHz, $CDCl_3$, δ en ppm, J en Hz) : 0,88 (s, 3H) ; 1,10 (s, 3H) 1,50-2,50 (m, H) ; 3,32 (d, J = 15,1H) ; 3,82 (d, J = 15,1H) ; 8,10 (s, 1H) ; 8,15 (s, 1H) ; 10,0 (s, 1H).
- spectre de résonance magnétique nucléaire du carbone (50 MHz, $CDCl_3$, δ en ppm) : 19,35 ; 25,1 ; 26,8 ; 42,1 ; 42,6 ; 48,4 ; 54,6 ; 58,8 ; 122,8 ; 138,4 ; 142,4 ; 184,6 ; 212,9.

Le 1H-imidazolecarboxaldéhyde-4 peut être préparé selon R.A. Turner et coll., J. Amer. Chem. Soc, 71, 2801 (1949).

## Revendications

1 - Procédé de préparation du racémique thréo du produit de formule :

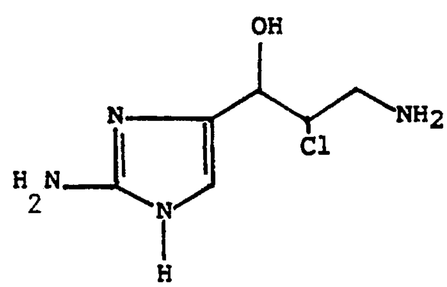

éventuellement sous forme de sel caractérisé en ce que l'on réduit un dérivé azo du racémique thréo de formule générale :

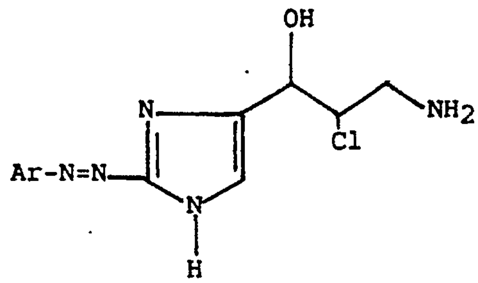

dans laquelle Ar représente un radical phényle éventuellement substitué par un ou plusieurs atomes ou radicaux, identiques ou différents, choisis parmi les atomes d'halogène et les radicaux alcoyles contenant 1 à 4 atomes de carbone ou alkoxycarbonyles dont la partie alkyle contient 1 à 4 atomes de carbone, ou nitro, et isole le produit obtenu éventuellement sous forme de sel.

2 - Procédé selon la revendication 1 caractérisé en ce que la réduction est effectuée au moyen d'hydrogène en présence d'un catalyseur d'hydrogénation.

3 - Procédé selon la revendication 2 caractérisé en ce que le catalyseur est l'oxyde de platine.

4 - Procédé selon l'une des revendications 1, 2 ou 3 caractérisé en ce que l 'on opéra dans un solvant organique.

5 - Procédé selon la revendication 4 caractérisé en ce que le solvant organique est choisi parmi les alcools.

6 - Procédé selon la revendication 5 caractérisé en ce que l'alcool est choisi parmi le méthanol et l'éthanol.

7 - Procédé selon l'une des revendications 1, 2, 3, 4, 5 ou 6 caractérisé en ce que l'on opère à une température comprise entre 0 et 30 ° C.

8 - L'amino-3 (amino-2 1H-imidazolyl-4)-1 chloro-2 propanol-1 et ses sels sous forme racémique thréo obtenu selon le procédé de l'une des revendications 1 à 7.

9 - Composition pharmaceutique caractérisée en ce qu'elle contient une quantité suffisante d'amino-3 (amino-2 1H-imidazolyl-4)-1 chloro-2 propanol-1 ou d'un de ses sels sous forme racémique thréo obtenu selon le procede de l'une des revendications 1 à 7.

Office européen des brevets

# RAPPORT DE RECHERCHE EUROPEENNE

Numero de la demande

EP 90 40 1237

| DOCUMENTS CONSIDERES COMME PERTINENTS | | | | |
|---|---|---|---|---|
| Catégorie | Citation du document avec indication, en cas de besoin, des parties pertinentes | Revendication concernée | CLASSEMENT DE LA DEMANDE (Int. Cl.5) | DOMAINES TECHNIQUES RECHERCHES (Int. Cl.5) |
| D,A | FR-A-2 585 020 (RHONE-POULENC-SANTE) | | C 07 D 233/88<br>A 61 K 35/56<br>A 61 K 31/415 | C 07 D 233/00<br>A 61 K 35/00<br>A 61 K 31/00 |

Le présent rapport a été établi pour toutes les revendications

| Lieu de la recherche | Date d'achèvement de la recherche | Examinateur |
|---|---|---|
| LA HAYE | 26-07-1990 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant